(19) Europäisches Patentamt / European Patent Office / Office européen des brevets

(11) **EP 1 737 272 B1**

(12) **FASCICULE DE BREVET EUROPEEN**

(45) Date de publication et mention
de la délivrance du brevet:
**08.09.2010   Bulletin 2010/36**

(51) Int Cl.:
*H05B 3/60* (2006.01)        *H05B 3/06* (2006.01)

(21) Numéro de dépôt: **06290993.2**

(22) Date de dépôt: **19.06.2006**

(54) **Cellule de chauffage ohmique**

Widerstandsheizzelle

Resistive heating cell

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **20.06.2005   FR 0506224**

(43) Date de publication de la demande:
**27.12.2006   Bulletin 2006/52**

(73) Titulaire: **Electricité de France
75008 Paris (FR)**

(72) Inventeurs:
  • **Berthou, Marc
    77670 Saint Mammes (FR)**
  • **Aussudre, Christian
    77210 Avon (FR)**
  • **Municino, Francesco
    29010 Rottofreno (Piacenza) (IT)**
  • **Massa, Mario
    42100 Reggio Emilia (IT)**

(74) Mandataire: **Boire, Philippe Maxime Charles et al
Cabinet Plasseraud
52 rue de la Victoire
75440 Paris Cedex 09 (FR)**

(56) Documents cités:
  WO-A-93/04421        WO-A-03/103340
  US-A- 5 609 900

**Description**

La présente invention concerne un dispositif de chauffage du type comprenant au moins deux électrodes raccordables à une source de tension et des moyens pour générer un écoulement continu du fluide le long d'un trajet passant entre les deux électrodes, notamment pour le traitement thermique de produits agroalimentaires fluides.

Afin de convenir aux différentes normes d'hygiène et de qualité, de nombreux produits agroalimentaires doivent subir un traitement physique et/ou chimique. Ainsi, la stérilisation de certains produits fluides est généralement obtenue par chauffage. Il est courant d'effectuer cette opération de chauffage de contact avec le fluide à chauffer.

Cependant, ce type de dispositif présente l'inconvénient d'un fort encrassement au niveau de la surface de contact, par exemple dans le cas de traitement de produits laitiers (boissons, extraits végétaux). Cela pose un problème sanitaire, mais également de rendement, puisque le fluide n'est plus en contact direct avec la surface de l'échangeur. Il est donc impératif d'interrompre le fonctionnement du dispositif de manière régulière afin de le nettoyer.

De plus, une inhomogénéité du traitement est caractéristique de ce type de chauffage puisque la température du fluide en tous points du dispositif n'est pas maîtrisée et dépend également du taux d'encrassement de la surface de l'échangeur. En outre, pour obtenir un chauffage plus homogène, il faut induire dans le fluide des mouvements de convection d'où une perte d'énergie.

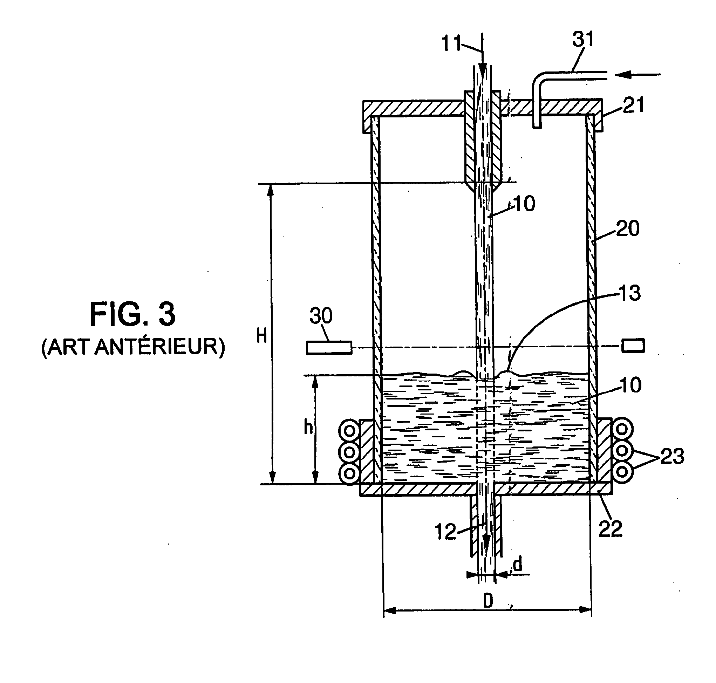

Dans le cas d'un dispositif du type mentionné en introduction, la résistance interne du fluide permet de transformer directement l'énergie électrique en énergie thermique par effet Joule. La figure 3 illustre un dispositif connu de ce type. Ce dispositif comprend une électrode de phase 21, en forme de buse d'injection au niveau de l'entrée 11 du fluide 10, et une électrode de masse 22, formant le fond du dispositif, au niveau de la sortie 12 du fluide 10. Une paroi extérieure 20 en matériau isolant sépare les deux électrodes. Un circuit de refroidissement 23 régule la température de l'électrode de masse 22. Un détecteur de niveau 30 fonctionnant par exemple à l'aide d'un faisceau laser, agit sur une injection d'azote 31, faisant ainsi varier la pression interne du dispositif, ce qui permet de régler le débit du fluide.

Les électrodes 21 et 22 sont raccordées à une source de tension, par exemple une source alternative de 1000 V avec une fréquence de 25kHz. Par conséquent, un courant alternatif circule dans la portion du fluide comprise entre les deux électrodes. Du fait de la résistance interne du fluide 10, celui-ci s'échauffe rapidement. Cette résistance est fonction du volume de fluide traversé. Ainsi, dans le cas précédent, on a :

$$R = K . [h/D^2 + (H-h)/d^2],$$

où R est la résistance du fluide, K est proportionnelle à la résistivité du fluide 10, H la distance séparant les électrodes 21 et 22, h le niveau de fluide 10 au fond du dispositif, D le diamètre du dispositif et d le diamètre du jet de fluide 10. La puissance transférée au fluide est directement fonction de cette résistance, égale à :

$$P = U^2/R,$$

où U est la tension entre les deux électrodes. Le détecteur de niveau 30 contrôle que le niveau de fluide h ne dépasse pas une valeur prédéterminée. Pour cela, il agit sur l'injection d'azote gazeux 31 de manière à faire varier la pression dans le dispositif, provoquant une variation du débit de sortie.

Un tel dispositif permet un traitement quasiment uniforme du fluide 10 et en continu. De plus, le chauffage est extrêmement rapide. Le contact réduit avec les parois permet de limiter les pertes d'énergie au travers des parois et procure un rendement supérieur à ce dispositif.

Cependant, un fort encrassement se développe sur l'électrode de masse 22 au fond du dispositif, ce qui impose un arrêt régulier du dispositif pour le nettoyer. Ensuite, un asservissement en température de ce dispositif est rendu difficile par les différentes non-linéarités de la puissance de chauffage transmise au fluide. Cette puissance est fonction du taux d'encrassement de l'électrode de masse, mais il est très difficile d'estimer cet encrassement. En outre, une inhomogénéité de température persiste dans la partie basse du dispositif, puisque le fluide 10 à proximité des parois extérieures est évacué moins rapidement et est donc traversé par le courant électrique plus longtemps. De plus, la perturbation 13 de la surface du liquide peut engendrer une erreur du détecteur de niveau 30, induisant une erreur de commande de la régulation.

Un but de la présente invention est de pallier ces différents inconvénients. Pour cela, l'invention propose un dispositif de chauffage d'un fluide en continu, dans lequel un courant traverse le fluide à chauffer.

L'invention propose donc un dispositif de chauffage comprenant au moins deux électrodes raccordables à une source de tension et des moyens pour générer un écoulement continu du fluide le long d'un trajet passant entre les deux électrodes, caractérisé en ce que l'une au moins des électrodes s'étend transversalement au trajet d'écoulement et comporte une portion renflée placée sur ledit trajet de façon que le fluide s'écoule autour de ladite portion renflée.

Conformément à un aspect de l'invention, la portion renflée est ovoïde.

Selon un autre aspect de l'invention, le dispositif comporte avantageusement une ou plusieurs des caractéristiques suivantes :

■ la portion renflée est sensiblement sphérique, et l'électrode comportant la portion renflée est agencée pour être entraînée en rotation autour de son axe transversal au trajet d'écoulement.

■ le dispositif comprend en outre des moyens de désencrassement d'une surface extérieure de ladite portion renflée de l'électrode, comme par exemple une racle en contact avec au moins une portion de la surface extérieure de la portion renflée, ou un générateur d'ultrasons.

■ un circuit de refroidissement est compris à l'intérieur de l'électrode comportant ladite portion renflée.

■ Le dispositif comprend en outre une paroi extérieure entourant de manière espacée la portion renflée de l'électrode, la paroi extérieure pouvant être en matériau isolant thermiquement et électriquement.

■ Le dispositif comporte également une entrée de fluide à chauffer entrant avec un débit d'entrée et une sortie de fluide chauffé sortant avec un débit de sortie, des moyens de régulation du débit d'entrée et un détecteur de niveau, dans lequel la sortie de fluide et le détecteur de niveau sont placés en aval de l'électrode comportant ladite portion renflée le long du trajet d'écoulement, et dans lequel le détecteur de niveau transmet un signal fonction du niveau de fluide en sortie aux moyens de régulation de débit d'entrée.

D'autres particularités et avantages de la présente invention apparaîtront dans la description ci-après d'exemples de réalisation non limitatifs, en référence aux dessins annexés, dans lesquels :

- la figure 1 est un schéma représentant un premier mode de réalisation, conforme à l'invention ;

- la figure 2 est un schéma représentant un deuxième mode de réalisation, conforme à l'invention ;

- la figure 3, déjà décrite, représente un dispositif conforme à l'état de la technique.

Sur les différentes figures, on a conservé les mêmes références pour les éléments identiques ou équivalents.

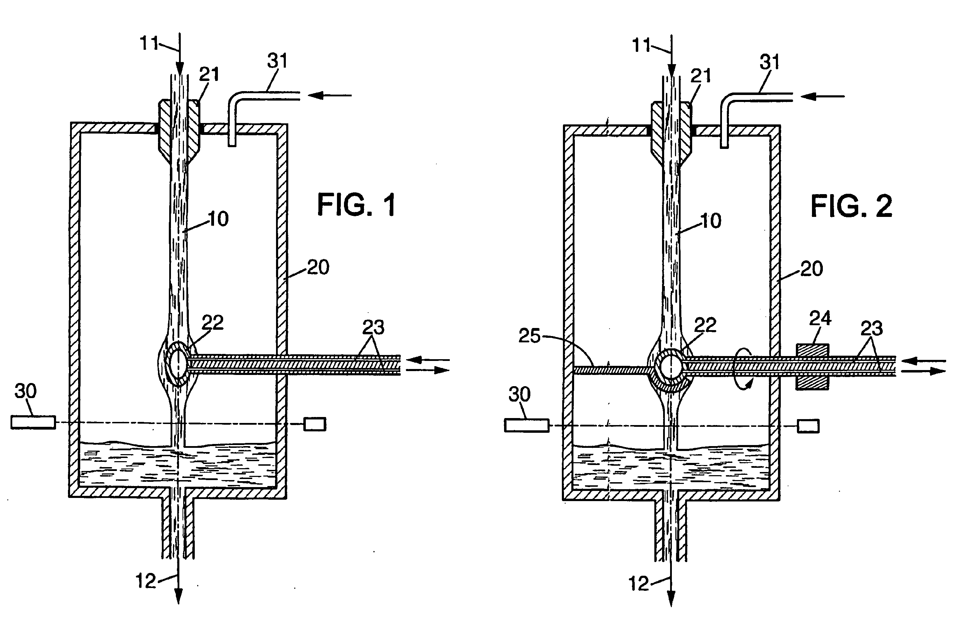

La figure 1 représente un dispositif selon un premier mode de réalisation. Il comprend une paroi extérieure 20, une électrode de phase 21 en forme de buse et une électrode de masse 22 dont une portion est de forme ovoïde. Un circuit de refroidissement 23 est compris dans l'électrode de masse 22. Un détecteur de niveau 30 permet de contrôler le niveau de fluide 10 dans la partie inférieure du dispositif. Un orifice d'injection d'azote 31 permet d'augmenter la pression à l'intérieur du dispositif de manière à réguler le débit à la sortie 12 du dispositif.
Le fluide 10 à chauffer pénètre dans le dispositif par l'entrée 11. Puis, il est dirigé par l'électrode de phase 21 en forme de buse sur l'électrode de masse 22. En raison de la différence de potentiel entre l'électrode de phase 21 et l'électrode de masse 22, un courant circule dans la portion de fluide comprise entre ces deux éléments. Le fluide 10 s'écoule autour de la partie ovoïde de l'électrode de masse 22 puis s'écoule vers le fond du dispositif. Le détecteur de niveau 30 contrôle que le niveau de fluide 10 n'atteint pas l'électrode de masse 22. Si le niveau augmente trop, le détecteur de niveau 30 envoie un signal à un dispositif d'injection d'azote 31 pour faire augmenter la pression interne du dispositif et ainsi augmenter le débit de sortie du fluide 10.
Ce mode de réalisation a l'avantage de simplifier la régulation en température du fluide. En effet, la résistance de la section de fluide à chauffer est sensiblement constante puisque son diamètre est constant. Pour une tension donnée, la puissance transférée au fluide lors de son passage entre les deux électrodes ne dépend que du débit d'entrée du fluide 10. Pour cette raison, le détecteur de niveau 30 ne requiert pas une grande précision puisqu'il ne fait que contrôler que le niveau de fluide 10 au fond du dispositif ne dépasse pas le niveau de l'électrode de masse 22. En outre, l'encrassement sur l'électrode de masse 22 est diminué en raison de sa forme. Enfin, l'homogénéité de traitement est accrue puisque la vitesse d'écoulement du fluide 10 est sensiblement uniforme entre les deux électrodes.
Afin de limiter encore l'encrassement des électrodes, un second mode de réalisation illustré à la figure 2 est proposé. Dans ce mode de réalisation, l'électrode de masse 22 est sphérique. Elle est mise en rotation autour de son axe transversal au trajet de fluide par un moteur 24. Une racle 25 est en contact avec la surface inférieure de la portion sphérique de l'électrode de masse 22 en raison de la rotation de l'électrode de masse 22, la racle frotte sur la surface extérieure de la portion sphérique de l'électrode de masse supprimant ainsi tout résidu de fluide qui pourrait provoquer un encrassement.
Ainsi, en gardant le rendement élevé du mode de réalisation précédent, ce mode de réalisation permet de limiter encore l'encrassement du dispositif et de simplifier la régulation en température de ce dispositif. Par conséquent, les interruptions pour nettoyer le dispositif sont limitées et ce dispositif peut fonctionner en continu pendant une plus longue durée.
Ainsi, tout en augmentant le rendement, cette invention permet également de maîtriser la formation d'encrassement sur les électrodes. En outre, ce dispositif ne comprend pas de paroi chaude et permet d'atteindre une rapidité de traitement ainsi qu'une plage de température élevée par rapport au dispositif avec échangeur thermique.

Selon un autre aspect de l'invention, des moyens de désencrassement peuvent également comprendre un générateur d'ultrasons placé sur l'électrode de masse.

Selon un autre aspect de l'invention, le dispositif est démontable et peut être modulé afin de changer des buses plus adaptées à d'autres débits.

**Revendications**

1. Dispositif de chauffage d'un fluide (10) comprenant au moins deux électrodes (21, 22) raccordables à une source de tension et des moyens (21) pour générer un écoulement continu du fluide le long d'un trajet passant entre les 2 électrodes, **caractérisé en ce que** l'une au moins des électrodes (22) s'étend transversalement au trajet d'écoulement et comporte une portion renflée placée sur ledit trajet de façon que le fluide (10) s'écoule autour de ladite portion renflée.

2. Dispositif selon la revendication 1, dans lequel ladite portion renflée est ovoïde.

3. Dispositif selon la revendication 1, dans lequel ladite portion renflée est sensiblement sphérique, et l'électrode (22) comportant ladite portion renflée est agencée pour être entraînée en rotation autour de son axe transversal au trajet d'écoulement.

4. Dispositif selon l'une quelconque des revendications précédentes, comprenant en outre des moyens (25) de désencrassement d'une surface extérieure de ladite portion renflée de l'électrode (22).

5. Dispositif selon la revendication 4, dans lequel les moyens de désencrassement comprennent une racle (25)en contact avec au moins une portion de la surface extérieure de ladite portion renflée de l'électrode (22).

6. Dispositif selon la revendication 4, dans lequel les moyens de désencrassement comprennent un générateur d'ultrasons.

7. Dispositif selon l'une quelconque des revendications précédentes, dans lequel un circuit de refroidissement (23) est compris à l'intérieur de l'électrode (22) comportant ladite portion renflée.

8. Dispositif selon l'une quelconque des revendications précédentes, comprenant en outre une paroi extérieure (20) entourant de manière espacée la portion renflée de l'électrode (22).

9. Dispositif selon la revendication 8, dans lequel la paroi extérieure (20) est en matériau isolant thermiquement et électriquement.

10. Dispositif de chauffage ohmique selon l'une quelconque des revendications précédentes, comportant une entrée (11) de fluide à chauffer entrant avec un débit d'entrée et une sortie (12) de fluide chauffé sortant avec un débit de sortie, des moyens de régulation du débit de sortie (31) et un détecteur de niveau (30), dans lequel la sortie de fluide (12)et le détecteur de niveau (30)sont placés en aval de l'électrode (22) comportant ladite portion renflée le long du trajet d'écoulement, et dans lequel le détecteur de niveau (30) transmet un signal fonction du niveau de fluide (10) en sortie aux moyens de régulation de débit de sortie.

**Claims**

1. Resistive heating cell for heating a fluid (10) comprising at least two electrodes (21, 22) connectable to a voltage source and means (21) to generate a continuous flow of fluid along a path passing between the 2 electrodes, **characterised in that** at least one of the electrode (22) extends transversally to the flow path and has a bulging portion located on the said path so that the fluid (10) flows around the said bulging portion.

2. Resistive heating cell according to Claim 1, wherein the said bulging portion is ovoid.

3. Resistive heating cell according to Claim 1, wherein the said bulging portion is substantially spherical, and the electrode (22) having the said bulging portion is adapted to be driven in rotation around its transversal axis to the flow path.

4. Resistive heating cell according to any one of the previous Claims further comprising unclogging means (25) of an outer surface of the said bulging portion of the electrode (22).

5. Resistive heating cell according to Claim 4, wherein the unclogging means comprise a scraper (25) being in contact with at least one portion of the outer surface of the said bulging portion of the electrode (22).

6. Resistive heating cell according to Claim 4, wherein the unclogging means comprise an ultrasonic generator.

7. Resistive heating cell according to any one of the previous Claims, wherein a cooling circuit (23) is comprised within the electrode (22) comprising the said bulging portion.

**8.** Resistive heating cell according to any one of the previous Claims further comprising an outer wall (20) surrounding in a spaced way the bulging portion of the electrode (22).

**9.** Resistive heating cell according to Claim 8 wherein the outer wall (20) is in a material insulating both thermally and electrically.

**10.** Ohmic resistive heating cell according to any one of the previous Claims, comprising an inlet (11) of fluids to be heated entering with a flow inlet and an outlet (12) of heated fluid exiting with an outlet flow, means for controlling the outlet flow (31) and a level sensor (30), wherein the outlet fluid (12) and the level detector (30) are placed downstream of the electrode (22) comprising the said bulging portion along the flow path, and wherein the level sensor (30) transmits an operation signal of the fluid level (10) in outlet to the means of control of the outlet flow.

**Patentansprüche**

**1.** Vorrichtung zum Heizen eines Fluids (10) umfassend mindestens zwei Elektroden (21, 22), die an eine Spannungsquelle anschließbar sind, und Mittel (21) zum Erzeugen eines kontinuierlichen Flusses des Fluids entlang eines Weges, der zwischen den 2 Elektroden verläuft, **dadurch gekennzeichnet, dass** mindestens eine der Elektroden (22) sich transversal zu dem Weg des Flusses erstreckt und einen bauchigen Abschnitt beinhaltet, der auf dem Weg derart angeordnet ist, dass das Fluid (10) um den bauchigen Abschnitt herum fließt.

**2.** Vorrichtung nach Anspruch 1, in der der bauchige Abschnitt eiförmig ist.

**3.** Vorrichtung nach Anspruch 1, in der der bauchige Abschnitt etwa kugelförmig ist, und die Elektrode (22), die den bauchigen Abschnitt beinhaltet, ist ausgestaltet, um rotatorisch angetrieben zu werden um seine transversale Achse zum Weg des Flusses.

**4.** Vorrichtung nach irgendeinem der vorhergehenden Ansprüche, des Weiteren umfassend Mittel (25) zum Entfetten einer äußeren Oberfläche des bauchigen Abschnitts der Elektrode (22).

**5.** Vorrichtung nach Anspruch 4, in der die Mittel zum Entfetten eine Rakel (25) umfassen, die in Kontakt ist mit mindestens einem Abschnitt der äußeren Oberfläche des bauchigen Abschnitts der Elektrode (22).

**6.** Vorrichtung nach Anspruch 4, in der die Mittel zum Entfetten einen Ultraschallgenerator umfassen.

**7.** Vorrichtung nach irgendeinem der vorhergehenden Ansprüche, in der ein Kühlkreislauf (23) im Inneren der Elektrode (22), die den bauchigen Abschnitt beinhaltet, enthalten ist.

**8.** Vorrichtung nach irgendeinem der vorhergehenden Ansprüche, des Weiteren umfassend eine äußere Wand (20), die den bauchigen Abschnitt der Elektrode (22) in beabstandeter Weise umgibt.

**9.** Vorrichtung nach Anspruch 8, in der die äußere Wand (20) aus thermisch und elektrisch isolierendem Material ist.

**10.** Vorrichtung zum Ohmschen Heizen nach irgendeinem der vorhergehenden Ansprüche, umfassend einen Einlass (11) des zu heizenden Fluids, das mit einem Einlassdurchfluss eintritt, und einen Auslass (12) des geheizten Fluids, das mit einem Auslassdmchfluss austritt, Mittel zum Regulieren des Auslassdurchflusses (31) und einen Füllstandsdetektor (30), in der der Auslass des Fluids (12) und der Füllstandsdetektor (30) flussabwärts der Elektrode (22), die den bauchigen Abschnitt beinhaltet, entlang des Weges des Flusses angeordnet sind, und in der der Füllstandsdetektor (30) ein Signal entsprechend des Füllstandes des ausfließenden Fluids (10) zu den Mitteln zum Regulieren des Auslassdurchflusses überträgt.

FIG. 1

FIG. 2

FIG. 3
(ART ANTÉRIEUR)

EP 1 737 272 B1